# EUROPÄISCHE PATENTSCHRIFT

(11) **EP 0 806 884 B1**
(45) Veröffentlichungstag und Bekanntmachung des Hinweises auf die Patenterteilung: **17.09.2003**
(21) Anmeldenummer: 97107228.5
(22) Anmeldetag: 30.04.1997
(51) Int. Cl.: H04R 25/00, A61B 5/12, A61F 11/00

(54) **Gerät zur elektromechanischen Stimulation und Prüfung des Gehörs**
Apparatus for the electro-mechanical stimulation and for the examination of the ears
Dispositif destiné à la stimulation électro-mécanique et à l'examen de l'ouie

(30) Priorität: 10.05.1996 DE 19618961
(43) Veröffentlichungstag der Anmeldung: 12.11.1997
(73) Patentinhaber: PHONAK AG, 8712 Stäfa (CH)
(72) Erfinder: Leysieffer, Hans, Dr. Dipl. Ing., 82024 Taufkirchen (DE)
(74) Vertreter: Schwan, Gerhard, Dipl.-Ing.

(56) Entgegenhaltungen:
- EP-A- 0 263 254
- DE-A- 3 121 429
- GB-A- 1 354 125
- US-A- 2 164 121
- US-A- 3 882 285
- HNO, Bd. 41, Nr. 1, Januar 1993, Seiten 1 -6, XP000603037 N. TASCHE U.A.: "LASER-DOPPLER-VIBROMETRIE (LDV) des TROMMELFELLS"

## Beschreibung

Die vorliegende Erfindung befaßt sich mit Geräten zur elektromechanischen Stimulation und Prüfung des Gehörs.

Geräte dieser Art und gemäβ dem Oberbegriff des Anspruchs 1 sind zum Beispiel aus der US-A-2 164 121 bekannt.

Generell wird das Hörvermögen des Menschen derart überprüft, daß ein Schallsignal und damit eine akustische Welle über geeignete elektroakustische Einrichtungen dem Probanden monaural (einohrig) bzw. binaural (beidohrig) dargeboten werden und der Proband auf entsprechende Fragestellungen subjektiv reagiert, die dem jeweiligen Zweck der psychoakustischen Untersuchung adäquat sind. Diese elektroakustischen Einrichtungen werden mit dem Sammelbegriff Audiometer bezeichnet, wobei in den häufigsten Anwendungsfällen das Prüfsignal entweder auf elektronischem Weg erzeugt wird (analoge bzw. digitale Signalgeneratoren) oder von einem geeigneten Tonträger (Magnetband, Compact Disc etc.) entnommen wird. Diese Prüfsignale werden dem Probanden auf *akustischem* Weg zumeist über Lautsprecher unter sog. Freifeldbedingungen oder über speziell kalibrierte Meß-Kopfhörer dargeboten. In Sonderfällen werden diese akustischen Signale über kurze Schalleitungsschläuche und Ohrpaßstücke zum äußeren Gehörgang geleitet, wenn z.B. ein akustisch dicht abgeschlossenes Volumen vor dem Trommelfell für die spezielle Prüfung gefordert wird. Darüber hinaus existieren objektive Hörprüfungsverfahren (z.B. BERA: *Brainstem Evoked Response Audiometry),* bei denen akustisch evozierte neuronale Antworten über Hautelektroden abgenommen und entsprechend analysiert werden *(Böhme, G., Welzl-Müller,* K.: *Audiometrie: Hörprüfungen im* Erwachsenen- und Kindesalter". Verlag Hans Huber, Bern, 1988, ISBN: *3-456-81620-0).*

Bei allen Verfahren wird grundsätzlich ein akustisches Signal dargeboten, welches auf bekannte Weise das Trommelfell in mechanische Schwingungen versetzt, die über die Gehörknöchelchenkette des Mittelohres zum Innenohr fortgeleitet und dort in ein neuronales Reizmuster umgewandelt werden, welches zu einem Höreindruck führt.

Insbesondere bei objektiven Hörprüfungsverfahren (z.B. BERA) bestehen in der akustischen Anregungsart jedoch einige Nachteile, wie z.B. die durch die zumeist verwendeten elektrodynamischen oder elektromagnetischen Kopfhörer erzeugten magnetischen Felder. Diese magnetischen (Stör-)Felder führen zu Problemen bei der Vorverarbeitung und Analyse der von der Hautoberfläche des Kopfes elektrisch abgeleiteten evozierten Potentiale, die im nV-Bereich liegen können. Bei überschwellig monaural dargebotenen akustischen Signalen entsteht weiterhin bei mittleren bis höheren Schallpegeln das Problem des "Überhörens" des nicht untersuchten, kontralateralen Ohres durch akustische Schallabstrahlung des Kopfhörers bzw. durch Körperschall (Knochenleitung), was zur Notwendigkeit der akustischen Vertäubung (Maskierung) dieses kontralateralen Ohres führt. Dieser Effekt ist bei zahlreichen psychoakustischen Fragestellungen unerwünscht, jedoch unvermeidlich.

Neuere Ansätze von teil- bzw. vollimplantierbaren Hörgeräten, bei denen das (geschädigte) Gehör nicht mehr akustisch, sondern mechanisch durch direkte mechanische Ankopplung eines entsprechenden Wandlers an verschiedene Bereiche des Mittelohres stimuliert wird, führen zu der Notwendigkeit, dem zur Implantation anstehenden Probanden die zu erwartende Hörverbesserung bzw. Klangqualität präoperativ zu demonstrieren. Dies ist mit bekannten

Verfahren jedoch nichtinvasiv, d.h. ohne operativen Eingriff, nicht möglich.

Es ist eine Aufgabe der vorliegenden Erfindung die oben genannten Probleme durch einen grundsätzlich anderen Ansatz der Signaldarbietung zu umgehen bzw. weitgehend zu vermeiden oder im Beispiel der (teil)implantierbaren Hörgeräte eine präoperative audiologische

Diagnostik adäquat überhaupt zu ermöglichen.

Diese Aufgabe wird erfindungsgemäß grundsätzlich dadurch gelöst, daß die Prüf- bzw. Demonstrationssignale nicht auf akustischem Weg, sondern durch direkte mechanische Stimulation der vom äußeren Gehörgang her zugänglichen Gehörknöchelchenkette des Mittelohres dem Gehör dargeboten werden. Zu diesem Zweck wird ein Gerät zur elektromechanischen Stimulation und Prüfung des Gehörs geschaffen, welches einen elektromechanischen Wandler zum Erzeugen von mechanischen Schwingungen im Audiobereich sowie ein starres, mechanisches Koppelelement aufweist, um die mechanischen Schwingungen ohne operativen Eingriff durch den äußeren Gehörgang in direktem mechanischem Kontakt auf das Zentrum des Trommelfells und damit auf den Hammergriff der Gehörknöchelchenkette des Mittelohres zu übertragen, wobei der elektromechanische Wandler in Verbindung mit dem mechanishen Koppelelement so ausgefürt ist, daβ die erste mechanische Resonanzfrequenz am oberen Ende des spektralen Ubertragungsbereiches von ≥ 10KH₂ liegt.

Auf diese Weise lassen sichaufgrund der Breitbandigkeit kurze Einchwingzeiten erreichen.

In Anwendung der Erfindung wird durch einen entsprechend ausgebildeten HNO-Arzt der schwingende Teil des elektromechanischen Wandlers über ein geeignetes Koppelelement unter optischer Kontrolle und mittels geeigneter Positionier- und Fixiationswerkzeuge durch den äußeren Gehörgang im zentralen Bereich des Trommelfells, dem Umbo (Nabel des Trommelfells, mit welchem der Endpunkt des Hammergriffs verwachsen ist), abgesetzt, mechanisch in direkten Kontakt mit diesem Zentrum gebracht und in direktem mechanischem Kontakt gehalten. Auf diesem Weg werden die mechanischen Schwingungen des Wandlers unmittelbar und direkt mechanisch an die Kette der Gehörknöchelchen gekoppelt und zum Innenohr fortgeleitet, und führen so zu einem Höreindruck.

Weitere Ausgestaltungen der Erfindung ergeben sich aus den Unteransprüchen.

Insbesondere können zum Erzeugen von audiologischen Prüf- und Testsignalen elektronische Signalgeneratoren vorgesehen sein, mit welchen sich frei wählbare Signale erzeugen lassen, oder es können alternativ bzw. wahlweise mit Tonträgern wie z.B. Magnetbändern oder Compact Discs arbeitende Signalquellen benutzt werden. Unabhängig von der Art der Signalquelle sollte ein Verstärker mit treibender Endstufe zum Zuführen der audiologischen Prüfund Testsignale zu dem elektromechanischen Wandler vorgesehen sein.

Das Einbringen des erfindungsgemäßen Geräts läßt sich einfacher für die untersuchende Person und gleichzeitig sicherer für den Patienten gestalten, wenn der elektromechanische Wandler in einem in den Eingangsbereich des äußeren Gehörgangs einzubringenden Wandlergehäuse untergebracht ist, dessen geometrische Abmessungen so gewählt sind, daß die untersuchende Person auch unter Verwendung eines Mikroskopes freie Sicht auf das das Zentrum des Trommelfells mechanisch kontaktierende Wirkende des Koppelelementes behält.

Vorzugsweise ist das Koppelelement als stabförmig ausgeführtes, in axialer Richtung steifes Bauteil ausgeführt, dessen von dem Wandler abgewandtes Wirkende einen verletzungsfreien, mechanischen Kontakt zum Zentrum des Trommelfells gewährleistet, wobei es sich als besonders vorteilhaft erweist, wenn das Koppelelement manuell leicht biegbar gestaltet ist, so daß es den individuellen geometrischen Formen des äußeren Gehörgangs angepaßt werden kann.

Wenn das Koppelelement nicht mechanisch fest, sondern über eine mechanische Steckverbindung mit dem Wandler verbunden ist, lassen sich z. B. unterschiedliche Koppelelemente realisieren, die beispielsweise aus hygienischen Gründen leicht austauschbar und als Einwegartikel ausführbar sind.

Um femer eine von den individuellen Schwankungen der biologischen Lastimpedanz unabhängige Einprägung der Auslenkung des Wirkendes des Koppelelementes zu erreichen, ist der elektromechanische Wandler zweckmäßig so ausgeführt, daß seine mechanische Quellimpedanz im gesamten spektralen Übertragungsbereich deutlich größer ist als die mechanische Lastimpedanz, die durch das biologische System Trommelfell, Gehörknöchelchenkette und Innenohr gebildet wird.

Die Untersuchung läßt sich für den Patienten noch angenehmer gestalten, wenn der elektromechanische Wandler durch die konstruktive Gestaltung des Wandlergehäuses akustisch so gekapselt ist, daß das Schallsignal, das durch die schwingenden Wandlerstrukturen abgestrahlt wird, so minimiert ist, daß bei hohen Stimulationspegeln auf eine akustische Vertäubung des kontralateralen, nicht untersuchten Ohres verzichtet werden kann.

Der elektromechanische Wandler kann auf dem Prinzip der elektrodynamischen, elektromagnetischen, magnetostriktiven, kapazitiven oder piezoelektrischen Wandlung beruhen, wobei ein nach dem piezoelektrischen Prinzip arbeitender elektromechanischer Wandler bevorzugt wird, da sich auf diese Weise insbesondere bei Untersuchungsmethoden mit abgeleiteten evozierten Potentialen magnetische Streufelder gänzlich vermeiden lassen.

In weiterer Ausgestaltung der Erfindung ist das aus Signalquelle, Verstärker und elektromechanischem Wandler mit Koppelelement bestehende System als präoperatives Diagnose- und Demonstrationsgerät der zu erwartenden Übertragungsqualität bei der Applikation teil- und vollimplantierbarer Hörgeräte ausgeführt.

Um eine simultane, beidohrige Stimulation und Prüfung des Gehörs zu ermöglichen, kann das Gerät doppelt ausgeführt sein.

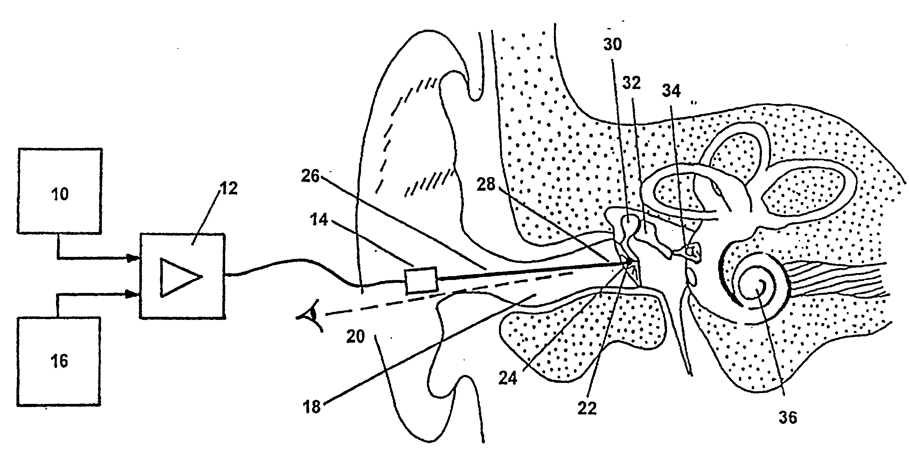

Die vorliegende Erfindung wird unter Bezugnahme auf die beiliegende einzige Figur, in welcher das erfindungsgemäße System im Detail dargestellt ist, näher beschrieben.

Unter Bezugnahme auf die Zeichnung werden von einem Generatorteil 10 elektrische Prüfsignale, wie z.B. reine Sinustöne oder breitbandige Signale (Rauschen etc.), erzeugt, wobei diese bezüglich der funktionalen Parameter (Frequenz, Pegel, zeitliche Sequenzen, Hüllkurven usw.) eingestellt werden können. Diese vorverarbeiteten Signale werden mit einem Verstärker 12, der eine treibende, dem gewählten Wandlerverfahren entsprechende Endstufe enthält, verstärkt und einem elektromechanischen Wandler 14 zugeführt. Alternativ zu der Erzeugung der Prüfsignale mittels des Generatorteils 10 können die Signale wie bei bekannten Audiometem auch von einem Tonträger 16 (Magnetband, Compact Disc etc.) entnommen und dem Verstärker 12 zugeführt werden. Der Wandler 14, der nach allen bekannten elektromechanischen Wandlungsprinzipien (dynamisch, magnetisch, piezoelektrisch, kapazitiv/dielektrisch, magnetostriktiv) arbeiten kann, wobei vorzugsweise die piezoelektrischen bzw. kapazitiven E-Feld-Wandlertypen aufgrund des Fehlens magnetischer Störfelder gewählt werden, wird mit geeigneten Hilfsmitteln nahe an den Eingang des äußeren Gehörgangs 18 des zu untersuchenden Ohres gebracht. Die äußeren Abmessungen dieses Wandlers 14 sind dabei so gestaltet, daß die untersuchende Person noch freien Einblick (gestrichelte Sichtlinie 20) durch den äußeren Gehörgang 18 bis auf das Zentrum 22 (Umbo) des Trommelfells 24 hat. Der elektromechanische Wandler 14 wandelt die elektrischen Treibersignale in mechanische Schwingungen um. Diese Wandlerschwingungen werden auf ein in seiner Längsachse mechanisch starres Koppelelement 26 übertragen, das mechanisch fest oder mittels einer (nicht gezeigten) Steckverbindung mit dem schwingenden Teil des Wandlers 14 verbunden ist. Das Koppelelement 26, das in der Zeichnung als stabförmiges Bauteil dargestellt ist, kontaktiert nach Absenken durch die untersuchende Person mit seinem dem Wandler abgewandten Ende 28 mechanisch mit leichtem Druck das Zentrum 22 (Umbo) des Trommelfells 24. Somit werden die mechanischen Wandlerschwingungen über die Gehörknöchelchenkette Hammer 30, Amboß 32 und Steigbügel 34 auf das Innenohr oder die Schnecke 36 übertragen, um zu einem Höreindruck zu führen. Das Ende 28 des stabförmigen Koppelelementes 26 ist dabei so gestaltet und durch geeignete Maßnahmen oberflächenbehandelt, daß einerseits nach Positionieren und Absenken ein Abrutschen vom Umbo 22 vermieden wird und andererseits eine Verletzungsgefahr dieses Trommelfellbereiches ausgeschlossen werden kann.

Nachdem die geometrischen Abmessungen des äußeren Gehörgangs 18 anatomisch individuellen Schwankungen unterliegen und der Gehörgang 18 grundsätzlich leicht gebogen verläuft, ist das stabförmige Koppelelement 26 vorzugsweise so gestaltet, daß es einerseits in axialer Richtung zur Vermeidung von mechanischen Resonanzen im Hörbereich eine möglichst hohe Steifigkeit aufweist und andererseits durch die untersuchende Person manuell ohne weiteres verformbar (biegbar) ist, um eine Anpassung an die leichte, individuelle Krümmung des Gehörgangs 18 zu crreichen und somit eine Berührung des schwingenden Koppelelementes mit Bereichen des äußeren Gehörgangs zu vermeiden. Wie oben erwähnt, ist es weiterhin zweckmäßig, wenn das Koppelelement 26 nicht fest mit dem Wandler 14 verbunden ist, sondern z.B. über eine mechanisch geeignete Steckvorrichtung. Auf diese Weise können unterschiedlich lange Koppelelemente für individuell verschiedene Längen des äußeren Gehörgangs realisiert werden, wobei diese unterschiedlichen Koppelelemente billig herstellbar sind und somit aus hygienischen Gründen bei Reihenuntersuchungen als EinmalArtikel ausführbar sind.

Erfindungsgemäβ ist das elektromechanische Gesamtsystem, bestehend aus Wandler 14 und Koppelelement 26, mit seinen das Betriebsverhalten bestimmenden dynamischen Komponenten Masse und Steifigkeit so dimensioniert, daß einerseits ein hochabgestimmtes System vorliegt, d.h., die erste mechanische Resonanzfrequenz am oberen Ende des angestrebten Übertragungsfrequenzbereiches (≥ 10 kHz) liegt. Durch diese Breitbandigkeit wird im Zeitverhalten eine kurze Einschwingzeit des Systems erreicht, was zu einem guten Impulsübertragungsverhalten des Systems führt. Andererseits sollte die mechanische Quellimpedanz dieses Systems deutlich über der biologischen Lastimpedanz liegen, die durch das System Trommelfell, Gehörknöchelchenkette und angekoppeltes, hydromechanisches Innenohr gebildet wird, um eine frequenzunabhängige Einprägung der Wandler- und damit Koppelelement-Auslenkung zu erreichen. Auf diese Weise ist es möglich, interindividuelle Hörprüfungsergebnisse zu vergleichen, da der Stimuluspegel dann nicht abhängig ist von der unbekannten, individuellen Schwankung der mechanischen (biologischen) Lastimpedanzen.

Die mit dem Gesamtsystem, bestehend aus der den Wandler 14 treibenden Endstufe und dem Wandler 14, erreichbare mechanische Auslenkung des Koppelelementes 26 sollte für audiologische subjektive und objektive Hörprüfungen Werte erreichen, die einem äquivalenten Schallpegel entsprechen, der am oberen Ende des audiologischen Dynamikbereiches liegt, also ca. 120 bis 130 dB SPL. Dies entspricht bei tiefen und mittleren Frequenzen bis ca. 1 - 2 kHz Auslenkungsamplituden von etwa 1 - 5 µm.

Bei entsprechender Ausführung des äußeren Wandlergehäuses (Kapselung) ist die unvermeidlich vorhandene akustische Schallabstrahlung der schwingenden Wandlerteile bei hohen Stimulationspegeln so reduzierbar, daß eine akustische, zusätzliche Anregung des untersuchten Ohres bzw. ein Mithören des kontralateralen Gehörs eliminiert wird, wodurch die Notwendigkeit der akustischen Vertäubung des kontralateralen Ohres vermieden wird.

Wird im Falle der Applikation eines teil- bzw. vollimplantierbaren Hörgerätes der elektromechanische Wandler dieses Implantatsystems als Wandler 14 und das signalvorverarbeitende Modul dieses Implantatsystems als Verstärker 12 verwendet, kann das erfindungsgemäße Gerät zur präoperativen Beurteilung der Übertragungsqualität und Tauglichkeit des vorgesehenen Implantatsystems bei dem betreffenden Probanden verwendet werden.

Weiterhin kann das erfindungsgemäße Gerät bei doppelter Ausführung beidohrig bei einem Probanden angewendet werden, um eine simultane, binaurale Hörprüfung durchzuführen.

## Patentansprüche

1. Gerät zur elektromechanischen Stimulation und Prüfung des Gehörs, **gekennzeichnet durch** einen elektromechanischen Wandler (14) zum Erzeugen von mechanischen Schwingungen im Audiobereich und ein starres, mechanisches Koppelelement (26) zum Übertragen der mechanischen Schwingungen ohne operativen Eingriff **durch** den äußeren Gehörgang (18) in direktem mechanischem Kontakt auf das Zentrum (22) des Trommelfells (24) und damit auf den Hammergriff der Gehörknöchelchenkette (30, 32, 34) des Mittelohres, **dadurch gekennzeichnet, daß** der elektromechanische Wandler (14) in Verbindung mit dem mechanischen Koppelelement (26) so ausgeführt ist, daß die erste mechanische Resonanzfrequenz am oberen Ende des spektralen Übertragungsbereiches von ≥ 10 kHz liegt.

2. Stimulations- und Prüfgerät nach Anspruch 1, **gekennzeichnet durch** mindestens einen elektronischen Signalgenerator (10) und/oder mindestens eine mit Tonträgern wie Magnetbändern oder Compact Discs arbeitende Signalquelle (16) zum Erzeugen von audiologischen Prüf- und Testsignalen sowie **durch** einen Verstärker (12) mit treibender Endstufe zum Zuführen der audiologischen Prüf- und Testsignale zu dem elektromechanischen Wandler (14).

3. Stimulations- und Prüfgerät nach einem der vorhergehenden Ansprüche, **dadurch gekennzeichnet, daß** der elektromechanische Wandler (14) in einem in den Eingangsbereich des äußeren Gehörgangs (18) einzubringenden Wandlergehäuse untergebracht ist, dessen geometrische Abmessungen so gewählt sind, daß die untersuchende Person auch unter Verwendung eines Mikroskopes freie Sicht auf das das Zentrum (22) des Trommelfells (24) mechanisch kontaktierende Wirkende (28) des Koppelelementes (26) behält.

4. Stimulations- und Prüfgerät nach einem der vorhergehenden Ansprüche, **dadurch gekennzeichnet, daß** das von dem Wandler (14) abgewandte Wirkende (28) des Koppelelements (26) konstruktiv so ausgebildet ist, daß ein verletzungsfreier, mechanischer Kontakt zum Zentrum (22) des Trommelfells (24) gewährleistet ist.

5. Stimulations- und Prüfgerät nach einem der vorhergehenden Ansprüche, **dadurch gekennzeichnet, daß** das Koppelelement (26) als stabförmiges, in axialer Richtung steifes Bauteil ausgeführt ist.

6. Stimulations- und Prüfgerät nach einem der vorhergehenden Ansprüche, **dadurch gekennzeichnet, daß** das Koppelelement (26) manuell leicht biegbar gestaltet ist.

7. Stimulations- und Prüfgerät nach einem der vorhergehenden Ansprüche, **dadurch gekennzeichnet, daß** das Koppelelement (26) über eine mechanische Steckverbindung mit dem Wandler (14) verbunden ist.

8. Stimulations- und Prüfgerät nach einem der vorhergehenden Ansprüche, **dadurch gekennzeichnet, daß** der elektromechanische Wandler (14) so ausgeführt ist, daß seine mechanische Quellimpedanz im gesamten spektralen Übertragungsbereich deutlich größer ist als die mechanische Lastimpedanz, die durch das biologische System Trommelfell (24), Gehörknöchelchenkette (30, 32, 34) und Innenohr (36) gebildet wird.

9. Stimulations- und Prüfgerät nach einem der Ansprüche 2 bis 8, **dadurch gekennzeichnet, daß** der den Wandler (14) treibende Verstärker (12) und der Wandler selbst so ausgelegt sind, daß der Wandler mit Koppelelement (26) im gesamten spektralen, audiologischen Übertragungsbereich bei mechanisch angekoppelter Gehörknöchelchenkette (30, 32, 34) maximale Auslenkungsamplituden im Bereich von 1-5 µm erzeugt, die einem äquivalenten Schalldruckpegel von 120 - 140 dB SPL entsprechen.

10. Stimulations- und Prüfgerät nach einem der vorhergehenden Ansprüche, **dadurch gekennzeichnet, daß** der elektromechanische Wandler (14) durch die konstruktive Gestaltung des Wandlergehäuses akustisch so gekapselt ist, daß das Schallsignal, das durch die schwingenden Wandlerstrukturen abgestrahlt wird, so minimiert ist, daß bei hohen Stimulationspegeln auf eine akustische Vertäubung des kontralateralen, nicht untersuchten Ohres verzichtet werden kann.

11. Stimulations- und Prüfgerät nach einem der vorhergehenden Ansprüche, **dadurch gekennzeichnet, daß** der elektromechanische Wandler (14) auf dem Prinzip der elektrodynamischen, elektromagnetischen, magnetostriktiven, kapazitiven oder piezoelektrischen Wandlung beruht.

12. Stimulations- und Prüfgerät nach Anspruch 11, **dadurch gekennzeichnet, daß** der elektromechanische Wandler (14) nach dem piezoelektrischen Prinzip arbeitet.

13. Stimulations- und Prüfgerät nach einem der Ansprüche 2 bis 12, **dadurch gekennzeichnet, daß** das aus Signalquelle (10, 16), Verstärker (12) und elektromechanischem Wandler (14) mit Koppelelement (26) bestehende System als präoperatives Diagnose- und Demonstrationsgerät der zu erwartenden Übertragungsqualität bei der Applikation teilund vollimplantierbarer Hörgeräte ausgeführt ist.

14. Stimulations- und Prüfgerät nach einem der vorhergehenden Ansprüche, **dadurch gekennzeichnet, daß** das Gerät doppelt ausgeführt ist, um eine simultane, beidohrige Stimulation und Prüfung des Gehörs zu ermöglichen.

## Claims

1. Device for electromechanical stimulation and testing of hearing, **characterized by** an electromechanical transducer (14) for generating mechanical vibrations in the audio range, and a rigid, mechanical coupling element (26) for transmitting the mechanical vibrations without surgery through the external auditory canal (18) of a patient in direct mechanical contact with the center (22) of the eardrum (24) for stimulation of the manubrium of malleus of the ossicular chain (30, 32, 34) of the middle ear, **characterized in that** the electromechanical transducer (14) in combination with the mechanical coupling element (26) is designed such that it has a first mechanical frequency of resonance which is at the upper end of the spectral transmission range of≥10 kHz.

2. Device for electromechanical stimulation and testing according to claim 1, **characterized by** at least one electronic signal generator (10) and / or at least one electronic signal source (16) that works with a playback device like magnetic tapes or compact disks for generating audiologic test signals, and an amplifier (12) with a driving end stage for delivering audiologic test signals to the electromechanical transducer (14).

3. Device for electromechanical stimulation and testing according to any one of the preceding claims, **characterized in that** the electromechanical transducer (14) is located in a transducer housing that is to be inserted into an inlet area of the external auditory canal (18) and that has geometrical dimensions selected such that the investigating person, even when using a microscope, has a free view of the active end (28) of the coupling element (26) which mechanically contacts the center (22) of the eardrum (24).

4. Device for electromechanical stimulation and testing according to any one of the preceding claims, **characterized by** that the active end (28) of the coupling element (26) which faces away from said transducer (14) is designed for enabling non-injurious mechanical contact with the center of the eardrum (24).

5. Device for electromechanical stimulation and testing according to any one of the preceding claims, **characterized in that** the coupling element (26) is a rod-like shaped component which is rigid in axial direction.

6. Device for electromechanical stimulation and testing according to any one of the preceding claims, **characterized in that** said coupling element (26) is manually easily bendable.

7. Device for electromechanical stimulation and testing according to any one of the preceding claims, **characterized in that** the coupling element (26) is joined to the transducer (14) by a mechanical plug-type connection.

8. Device for electromechanical stimulation and testing according to one of the preceding claims, **characterized in that** the electromechanical transducer (14) has a mechanical source impedance which is greater than the mechanical load impedance which is formed by a biological system consisting of the eardrum (24), ossicular chain (30, 32, 34) and inner ear (36) in the entire spectral transmission range.

9. Device for electromechanical stimulation and testing according any one of claims 2 - 8 , **characterized in that** the amplifier (12) driving the electromechanical transducer (14) and the transducer itself are constructed such that the transducer with coupling element (26) when mechanically coupled to the ossicular chain (30, 32, 34) generates maximum deflection amplitudes in the range of 1-5 µm, which correspond to an equivalent sound pressure level of 120-140 dB SPL, in the entire spectral audiologic transmission range.

10. Device for electromechanical stimulation and testing according to any one of the preceding claims, **characterized in that** the electromechanical transducer (14) is acoustically enclosed by a transducer housing in such a manner the acoustic signal emissions from vibrating transducer structures are mininmzed such that at high stimulation levels the need for acoustic masking of the contralateral ear not being tested is eliminated.

11. Device for electromechanical stimulation and testing according to any one of the preceding claims, **characterized in that** the electromechanical transducer (14) is based on the principle of electrodynamic, electromagnetic, magnetostrictive, capacitive, or piezoelectric conversion.

12. Device for electromechanical stimulation and testing according to claim 11, **characterized in that** the electromechanical transducer (14) is based on the principle of piezoelectric conversion.

13. Device for electromechanical stimulation and testing according to any one of claims 2-12, **characterized in that** the system consisting of signal source (10, 16), amplifier (12) and the electromechanical transducer (14) with the mechanical coupling element (26) is a preoperative diagnosis and demonstration device for demonstrating the expected transmission quality corresponding to that of applicable partially and fully implantable hearing aids.

14. Device for electromechanical stimulation and testing according to any one of the preceding claims, **characterized in that** the device is designed to be twofold for enabling simultaneous binaural stimulation and testing of hearing.

## Revendications

1. Appareil destiné à la stimulation électromécanique et à l'examen de l'ouïe, **caractérisé par** un convertisseur électromécanique (14) pour la génération de vibrations mécaniques dans la plage audio, et par un élément de couplage mécanique rigide (26) destiné à la transmission des vibrations mécaniques en contact mécanique direct, sans intervention opératoire par le conduit auditif externe (18), au centre (22) du tympan (24), et par conséquent au manche du marteau de la chaîne des osselets auditifs (30, 32, 34) de l'oreille moyenne, **caractérisé en ce que** le convertisseur électromécanique (14) en liaison avec l'élément de couplage mécanique (26) est réalisé de telle sorte que la première fréquence de résonance mécanique soit située au niveau de l'extrémité supérieure de la plage de transmission spectrale ≥ 10 kHz.

2. Appareil de stimulation et d'examen selon la revendication 1, **caractérisé par** au moins un générateur de signaux électronique (10) et/ou au moins une source de signaux (16) fonctionnant avec des supports d'enregistrement, tels que des bandes magnétiques ou des disques compacts, pour la génération de signaux audiologiques d'examen et de test, ainsi que par un amplificateur (12) à étage final d'attaque pour l'amenée des signaux audiologiques d'examen et de test au convertisseur électromécanique (14).

3. Appareil de stimulation et d'examen selon l'une quelconque des revendications précédentes, **caractérisé en ce que** le convertisseur électromécanique (14) est logé dans un boîtier de convertisseur à introduire dans la zone d'entrée du conduit auditif externe (18), dont les dimensions géométriques sont choisies de telle sorte que, même en utilisant un microscope, la personne effectuant l'examen ait une vue libre sur l'extrémité active (28) de l'élément de couplage (26) qui est en contact mécanique avec le centre (22) du tympan (24).

4. Appareil de stimulation et d'examen selon l'une quelconque des revendications précédentes, **caractérisé en ce que**, du point de vue constructif, l'extrémité active (28) de l'élément de couplage (26) opposée au convertisseur (14) est agencée de telle sorte qu'un contact mécanique sans lésions soit assuré avec le centre (22) du tympan (24).

5. Appareil de stimulation et d'examen selon l'une quelconque des revendications précédentes, **caractérisé en ce que** l'élément de couplage (26) est réalisé sous la forme d'un composant en forme de barre, qui est rigide dans la direction axiale.

6. Appareil de stimulation et d'examen selon l'une quelconque des revendications précédentes, **caractérisé en ce que** l'élément de couplage (26) est agencé de façon à pouvoir être facilement cintré manuellement.

7. Appareil de stimulation et d'examen selon l'une quelconque des revendications précédentes, **caractérisé en ce que** l'élément de couplage (26) est relié au convertisseur (14) par l'intermédiaire d'une connexion mécanique enfichable.

8. Appareil de stimulation et d'examen selon l'une quelconque des revendications précédentes, **caractérisé en ce que** le convertisseur électromécanique (14) est réalisé de telle sorte que, dans l'ensemble de la plage de transmission spectrale, son impédance de source mécanique soit nettement supérieure à l'impédance de charge mécanique, qui est constituée du système biologique formé par le tympan (24), la chaîne des osselets auditifs (30, 32, 34) et l'oreille interne (36).

9. Appareil de stimulation et d'examen selon l'une quelconque des revendications 2 à 8, **caractérisé en ce que** l'amplificateur (12) attaquant le convertisseur (14), et le convertisseur lui-même, sont conçus de telle sorte que, lorsque la chaîne des osselets auditifs (30, 32, 34) est couplée mécaniquement, le convertisseur avec l'élément de couplage (26) génère dans l'ensemble de la plage de transmission spectrale audiologique des amplitudes maximales de déviation se situant dans la plage de 1 à 5 µm, qui correspondent à un niveau de pression sonore équivalent de 120 à 140 dB SPL.

10. Appareil de stimulation et d'examen selon l'une quelconque des revendications précédentes, **caractérisé en ce que** le convertisseur électromécanique (14) est encapsulé du point de vue acoustique par l'agencement constructif du boîtier de convertisseur, de telle sorte que le signal sonore, qui est émis pas les structures vibrantes du convertisseur, soit minimisé à un point tel que, lors de niveaux de stimulation élevés, il peut être renoncé à une neutralisation acoustique de l'oreille controlatérale non examinée.

11. Appareil de stimulation et d'examen selon l'une quelconque des revendications précédentes, **caractérisé en ce que** le convertisseur électromécanique (14) est basé sur le principe de la conversion électrodynamique, électromagnétique, magnétostrictive, capacitive ou piézoélectrique.

12. Appareil de stimulation et d'examen selon la revendication 11, **caractérisé en ce que** le convertisseur électromécanique (14) fonctionne selon le principe piézoélectrique.

13. Appareil de stimulation et d'examen selon l'une quelconque des revendications 2 à 12, **caractérisé en ce que** le système constitué de la source de signaux (10, 16), de l'amplificateur (12) et du convertisseur électromécanique (14) avec élément de couplage (26) est réalisé en tant qu'appareil de diagnostic et de démonstration préopératoire de la qualité de transmission à attendre par l'implantation d'appareils auditifs partiellement et complètement implantables.

14. Appareil de stimulation et d'examen selon l'une quelconque des revendications précédentes, **caractérisé en ce que** l'appareil est réalisé en double afin de permettre une stimulation et un examen simultanés de l'ouïe des deux oreilles.
